# EUROPEAN PATENT SPECIFICATION

(11) **EP 1 568 365 B1**
(45) Date of publication and mention of the grant of the patent: **11.04.2018**
(21) Application number: 03775929.7
(22) Date of filing: 27.11.2003
(51) Int. Cl.: A61K 31/045, A61K 31/165, A61K 9/70, A61K 47/10, A61K 47/34, A61P 43/00

(54) **WARM POULTICE**
WARMER BREIUMSCHLAG
CATAPLASME CHAUD

(30) Priority: 27.11.2002 JP 2002344398
(43) Date of publication of application: 31.08.2005
(73) Proprietor: HISAMITSU PHARMACEUTICAL CO., INC., Tosu-shi, Saga-ken 841-0017 (JP)
(72) Inventor: MUTA, Kazunori, Tosu-shi Saga 841-0017 (JP); HINOTANI, Tomoyuki, Tosu-shi Saga 841-0017 (JP)
(74) Representative: Grünecker Patent- und Rechtsanwälte PartG mbB
(86) International application number: PCT/JP2003/015176
(87) International publication number: WO 2004/047820

(56) References cited:
- WO-A1-01/10435
- JP-A- 3 161 435
- US-B1- 6 469 227

## Description

### Technical Field

The present invention relates to a warming patch comprising a support and an adhesive layer.

### Background Art

In the prior art, a warming material containing a pharmaceutical agent such as capsaicin for giving a warm feeling to the adhesive layer of a patch, is described for example in JP-A 2000-143507, JP-A 06-256183, WO 01/10435 A1 and JP-A 10-298065. For this warming material to maintain the warm feeling sufficiently while the patch was in use, it was necessary to blend a relatively large amount of the material into the adhesive layer of the patch. However, when such a large amount of the warming material was blended into the adhesive layer in this way, after the patch was peeled off, there was a problem of residual skin irritation.

In particular, capsaicin left a strong residual irritation, and when a bath was taken after applying a patch containing capsaicin and water to the skin, the residual irritation (tingling feeling as if the skin was being pierced) was intolerable. For this reason, a capsaicin-containing, moisture-containing agent, which had a sufficient warming effect and a reduced residual irritation, was desired.

JP-A 11-199522 describes a topical skin preparation wherein a given amount of a cooling agent, 1-menthol, is blended with capsaicin. However, even when 1-menthol was blended with capsaicin, the problem of residual irritation due to capsaicin remained in topical skin preparations containing 1-menthol, and the residual irritation was tended to be actually worse, although the warming effect of capsaicin improved despite the cooling effect of 1-menthol. Thus, a warming patch wherein residual irritation was fully reduced had not yet been obtained.

US 6 469 227 B1 and JP-A 03-161435 disclose a menthol-containing medical patch and a cataplasm for improving the skin irritability, respectively.

### Disclosure of the Invention

It is therefore an object of the present invention, which was conceived in view of the aforesaid prior art, to provide a warming patch which has a sufficient warming effect, makes the pleasant stimulus due to the warming effect more long-lasting, and has a much reduced residual irritation.

As a result of intensive studies intended to achieve the aforesaid object, the Inventors discovered that when 1-menthol and polyethylene glycol are blended with the adhesive layer of the warming patch, the warming effect of a warming material such as capsaicin is improved despite the cooling effect of 1-menthol, and the pleasant feeling due to the moderate warming effect is more long-lasting due to the polyethylene glycol. More surprisingly, they discovered that although the aforesaid effect was obtained while the patch was in use, polyethylene glycol functions as a residual irritation-reducing agent which largely reduces the residual irritation due to the warming material such as capsaicin after the patch is peeled off, and thereby arrived at the present invention. It became clear that the blending of 1-menthol and polyethylene glycol with the adhesive layer of the warming patch was also excellent from the viewpoint of ease of manufacturing the adhesive layer.

The warming patch (warming, moisture-containing patch) of the present invention comprises a support and an adhesive layer arranged on at least one surface of this support, the adhesive layer containing a warming material, 1-menthol and polyethylene glycol as a residual irritation-reducing agent.

The warming material is selected from a group comprising capsaicin, dihydroxycapsaicin, capsanthin, capsaicinoid, capsicoside, capsicum extract, capsicum tincture, and capsici fructus pulveratus.

The warming patch of the present invention contains 0.01-0.03 mass % of the warming material, 0.5-1.5 mass % of 1-menthol and 5-15 mass % of polyethylene glycol, respectively, based on the whole amount of the adhesive layer.

The molecular weight of the polyethylene glycol may be in the range of 200-4000, and the adhesive layer may further contain a non-steroidal anti-inflammatory drug.

The support used in the warming patch of the present invention preferably has a triple layer structure comprising a thermoplastic resin film, and a fibrous sheet which may be a woven fabric or a nonwoven fabric disposed on both sides of this resin film.

The support preferably has a water vapor transmission (moisture permeability) of 500-6000 g/m²/24hr. By using a support having a moisture vapor transmission within the aforesaid range, the warming effect of the warming material can be adjusted to a suitable intensity, and the pleasant stimulation mentioned above can be further enhanced. The water vapor transmission (moisture permeability) can be measured by the method specified in JIS (Japan Industrial Standard) Z 0208.

### Brief Description of the Drawings

FIG. 1 is a schematic cross-sectional view showing one aspect of the warming patch of the present invention.
FIG. 2 is a schematic cross-sectional view showing another aspect of the warming patch of the present invention.

### Best mode for carrying out the Invention

Hereafter, a suitable aspect of the warming patch of the present invention will be described in detail referring to the accompanying drawings. In the drawings, identical or equivalent parts are assigned identical symbols and their description is not repeated.

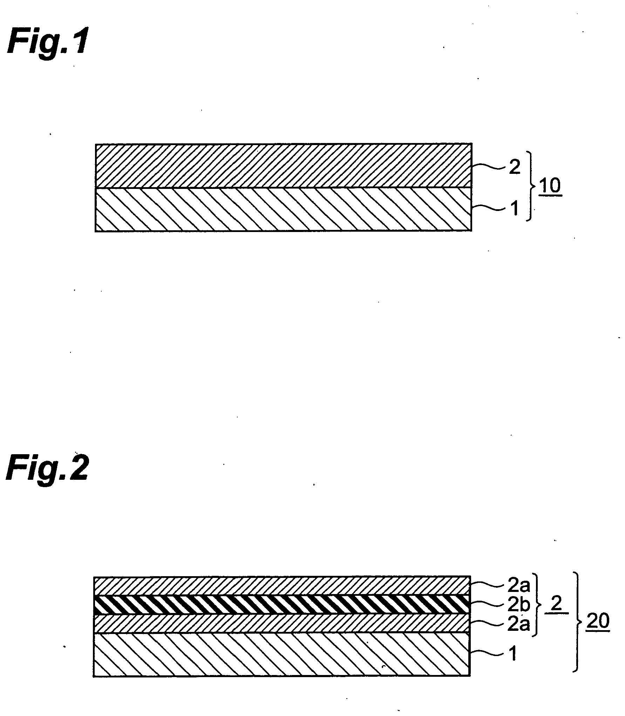

FIG. 1 is a schematic cross-sectional view showing one aspect of the warming patch of the present invention. A warming patch 10 shown in FIG. 1 comprises a support 2 and an adhesive layer 1 arranged on at least one surface of the support 2, the adhesive layer 1 containing a warming material, 1-menthol and polyethylene glycol as a residual irritation-reducing agent.

The warming material may be capsaicin (N-(4-hydroxy-3-methoxybenzyl)-8-methyl-6-nonene amide); a capsaicin analog, such as dihydroxycapsaicin, capsanthin, capsaicinoid and capsicoside; a warming material from capsici fructus, such as capsicum extract, capsicum tincture and capsici fructus pulveratus. One, two or more such materials may be used. A warming material from capsici fructus usually contains capsaicin. The warming material may form salts (sodium salt, potassium salt, ammonium salt, etc.).

Among the aforesaid warming material, capsaicin or warming material from capsici fructus is preferred.

By containing such a warming material in the adhesive layer 1 of the patch 10, the skin is stimulated, a warming effect is obtained, blood circulation is facilitated and a mild stimulus continues for a long time.

The blending amount of the warming material is 0.01 to 0.03 mass % based on the total amount of the adhesive layer 1. If the blending amount of the pharmaceutical agent is less than the aforesaid lower limit, the skin stimulation due to the warming material is not fully obtained, so the warming effect and facilitation of blood circulation tend to be incomplete, whereas if the aforesaid limit is exceeded, there is too much skin stimulation and marked residual irritation tend to remain after the patch is peeled off.

1-menthol which is used as a pharmaceutical agent is a monoterpene alcohol, and is a principal ingredient of peppermint oil. 1-menthol give skin a cool and fresh feeling, leaving the skin refreshed and invigorated after use, and the warming effect of the warming material can be further enhanced by using it together with the warming material.

The blending amount of 1-menthol is not particularly limited, but it is 0.5 to 1.5 mass % based on the total amount of the adhesive layer 1. If the blending amount of 1-menthol is less than the aforesaid lower limit, the synergistic improvement of the warming effect due to the warming material is insufficient, whereas if on the other hand, the aforesaid upper limit is exceeded, crystals may deposit over a long period of time and the cost may increase.

Polyethylene glycol is contained in the adhesive layer of the warming patch. Thus, it is one of the features of the present invention to use
polyethylene glycol as a residual irritation-reducing agent. Polyethylene glycol is a polymer of ethylene glycol and is classified as a polyether. In general, polyethylene glycol is manufactured by polymerization of ethylene oxide, and in general, there are hydroxyl groups at both ends of the molecule.

By containing polyethylene glycol in the adhesive layer 1 of the patch, the pleasant stimulation accompanying the moderate warming effect due to the warming material is even more long-lasting, and at the same time, the residual irritation due to the warming material can be largely reduced after peeling the patch off.

The polyethylene glycol preferably has an average molecular weight of 200-4000. The average molecular weight is more preferably 200-1500, still more preferably 200-600, and particularly preferably 200-500. If the average molecular weight is less than 200, the durability of the pleasant stimulation and the reduction of residual irritation are not fully obtained. On the other hand, if the aforesaid respective upper limits are exceeded, operability is impaired, while the durability of the pleasant stimulation and the reduction of residual irritation are not fully obtained. Polyethylene glycol can be obtained from Dai-Ichi Kogyo Seiyaku, Asahi Denka Kogyo, Sanyo Chemical Industries or Nippon Oil & Fats.

The blending amount of polyethylene glycol is 5 to 15 mass %, based on the total amount of the adhesive layer 1. If the blending amount is less than the aforesaid lower limit, the reduction of residual irritation after peeling the patch off, and particularly the prickling feeling by elevating body temperature due to bathing, etc., is inadequate. If the aforesaid upper limit is exceeded, the tackiness of the adhesive layer tends to be insufficient.

The adhesive layer 1 of the warming patch 10, in addition to the warming material, 1-menthol and polyethylene glycol as residual irritation-reducing agent, contains water and hydrophilic polymer such as a water-soluble polymer forming the skeleton of the patch, water-absorbing polymer or polymer which gels with water. It may also contain ingredients which are generally blended with the adhesive layer of a patch, such as a polyhydric alcohol, polyvalent metallic salt and surfactant.

The hydrophilic polymer which functions as the base of the adhesive layer may for example be gelatin, a polyacrylic acid, a salt thereof (metal salt, such as lithium, sodium or potassium salt) or partially neutralized substance, polyvinyl alcohol, polyacrylamide, polyethylene oxide, polyethylene imine, polyvinyl pyrrolidone, carboxyvinyl polymer, methyl cellulose, carboxymethylcellulose or hydroxyethylcellulose. These may be used alone, or two or more kinds thereof may be blended together. Among these, by using one, two or more of gelatin, polyacrylic acid, a salt thereof, a partially neutralized substance or polyvinyl alcohol, an adhesive material with good tackiness and shape-holding property can be obtained.

The blending amount of the hydrophilic polymer is preferably 5 to 25 mass %, more preferably 8 to 20 mass %, and still more preferably 10 to 15 mass %, based on the total amount of the adhesive layer 1. If the blending amount is less than the aforesaid lower limit, the tackiness is insufficient and the patch easily peels off. If on the other hand the aforesaid upper limit is exceeded, the tackiness is too strong, and there are problems in use such as pain when the patch is peeled off. The blending amount is preferably also determined considering the tackiness and cohesiveness of the pharmaceutical preparation, shape-holding property, water absorption capacity,
non-homogeneity of fat, operability decreases, unsatisfactory user experience and viscosity during manufacture.

The water contained in the adhesive layer may be purified water, sterilized water or natural water. Water acts as a dispersing agent and solvent of the hydrophilic polymer and other ingredients. In particular, it is important to uniformly-disperse and dissolve the glycol and polyhydric alcohol in the pharmaceutical preparation, described later; which act as moisturizers. Further, water itself remarkably enhances user experience during and after use, and diffuses into the skin together with moisturizing ingredients so as to leave the skin moist and firm.

The blending amount of water is preferably 30 to 95 mass %, but more preferably 40 to 80 mass %, and still more preferably 50 to 70 mass % based on the total amount of the adhesive layer 1. If the blending amount is less than the aforesaid lower limit, operability decreases and costs rise, whereas if on the other hand the aforesaid upper limit is exceeded, it tends to be difficult to maintain the shape-holding property. The blending amount is also determined considering the tackiness of the pharmaceutical preparation, loss of water retention prior to use, loss of operability, unsatisfactory user experience in use, and shape-holding property prior to use.

The aforesaid polyhydric alcohol may for example be glycerol, ethylene glycol, 1,3-butylene glycol, propylene glycol, dipropylene glycol, sorbitol or xylitol. Among these, glycerol is particularly preferred from the viewpoint of operability and user experience, etc.

The blending amount of polyhydric alcohol is preferably 5 to 45 mass %, more preferably 15 to 35 mass %, and still more preferably 20 to 30 mass % based on the total amount of the adhesive layer 1. If the blending
amount is less than the aforesaid lower limit, the moisture in the pharmaceutical preparation vaporizes easily when the patch is applied, and there is a tendency for tackiness to decrease and the patch to peel off easily. If on the other hand the aforesaid upper limit is exceeded, further enhancement of tackiness tends not to be obtained which is uneconomical.

The aforesaid polyvalent metallic salt may be for example aluminum hydroxide, aluminum hydroxide gel, hydrated aluminum silicate, synthetic aluminum silicate, kaolin, aluminum acetate, aluminum lactate, aluminum stearate, calcium chloride, magnesium chloride, aluminum chloride, magnesium aluminometasilicate or magnesium aluminum silicate. Among these, synthetic aluminum silicate and magnesium aluminometasilicate are particularly preferred.

The blending amount of polyvalent metallic salt is preferably 0.01 to 5 mass %, but more preferably 0.05 to 3 mass %, and still more preferably 0.1 to 2 mass % based on the total amount of the adhesive layer 1. If the blending amount is less than the aforesaid lower limit, the reaction does not proceed completely and the gel strength tends to be insufficient, whereas if on the other hand the aforesaid upper limit is exceeded, the reaction rate during manufacture is too rapid, gelling tends to be uneven and operability tends to be poor.

As a gelling rate adjusting agent, EDTA which has a chelating effect on metal ions, an organic acid such as acetic acid, lactic acid, oxalic acid, citric acid, and tartaric acid, an organic acid salt such as EDTA-disodium, calcium citrate, sodium citrate and disodium citrate, or an organic base, may also be blended.

The aforesaid surfactant may be an anionic surfactant, such as sodium dioctyl sulfosuccinate, an alkyl sulfate salt, 2-ethylhexyl alkyl sulfuric acid ester sodium salt and normal sodium dodecylbenzenesulfonate; a cationic surfactant, such as hexadecyltrimethyl ammonium chloride, octadecyl dimethylbenzylammonium chloride and polyoxyethylene dodecyl monomethylammonium chloride; or a non-ionic surfactant, such as polyoxyethylene stearyl ether, polyoxyethylene-hardened castor oil, polyoxyethylene tridecyl ether, polyoxyethylene nonylphenyl ether, polyoxyethylene octyl phenyl ether, polyoxyethylene monostearate, sorbitan monostearate, sorbitan monopalmitate, sorbitan sesquioleate, polyoxyethylene sorbitan monolaurate, polyoxyethylene sorbitan mono-oleate, glycerol monostearate, polyglyceryl fatty acid ester and polyoxyethylene octadecyl amine. Among these, polyoxyethylene monostearate and polyoxyethylene-hardened castor oil are particularly preferred.

The blending amount of surfactant is preferably 0.05 to 2 mass %, but more preferably 0.2 to 1.5 mass %, and still more preferably 0.4 to 1.0 mass % based on the total amount of the adhesive layer 1. If the blending amount is less than the aforesaid lower limit, it tends to cause bleeding, and if on the other hand the aforesaid upper limit is exceeded, it is difficult to maintain the shape-holding property.

In the present invention, it is also possible to blend a skin beautifying ingredient, non-steroidal anti-inflammatory drug, moisturizing ingredient, antioxidant crosslinking agent, antiseptic, tackifier, solvent, pigment, perfume, ultraviolet absorber, inorganic filler or pH adjuster if required.

The aforesaid skin beautifying ingredient may be for example a water-soluble placental extract, allantoin, lecithin, amino acid, kojic acid, kojic
acid derivative (preferably a kojic acid derivative to which an alkyl group having 5-20 carbon atoms is added), protein, hormone, crude drug extract (an extract such as aloe, sponge gourd and liquorice), seaweed extract, vitamin (vitamin A, vitamin C, vitamin D, vitamin E, etc), caffeine, diphenhydramine hydrochloride, diphenhydramine salicylate, diphenhydramine tannate, triprolidine hydrochloride, mequitazine, chlorpheniramine maleate, d-chlorpheniramine maleate, clemastine fumarate, promethazine hydrochloride, tranilast, sodium cromoglicate, ketotifen, arylsulfatase B, bufexamac, bendazac, butyl flufenamate, ibuprofen, indomethacin, aspirin, flurbiprofen, ketoprofen, piroxicam, 2-pyridine methyl phenamic acid, 5,6-dihydroarachidonic acid, esculetin, eupatilin, 4-dimethyleupatilin, caffeic acid or benoxaprofen, etc. Among these, water-soluble placental extract and allantoin are particularly preferred.

The aforesaid non-steroidal anti-inflammatory drug may be for example indomethacin, ketoprofen, felbinac, flurbiprofen, ketorolac, piroxicam, loxoprofen, ibuprofen piconol or sodium diclofenate, etc.

The aforesaid moisturizing ingredient may be acylated kefiran aqueous solution, malt extract or a glycol, used alone or in combination. The blending amounts thereof are not particularly limited, but are preferably 1 to 50 mass %, more preferably 5 to 30 mass %, and still more preferably 5 to 25 mass % based on the total amount of the adhesive layer 1. The blending amounts are preferably determined considering the decrease of tackiness and cohesiveness of the pharmaceutical preparation, decrease of water retention and shape-holding property before use, non-homogeneity of gel, loss of operability and poor user experience in use.

As the aforesaid glycol, polypropylene glycol of average molecular weight 500-3000 which is a glycol with a polyether structure, is preferred.

The aforesaid antioxidant may be for example ascorbic acid, propyl gallate, butylated hydroxyanisole, dibutylhydroxytoluene, nordihydroguaiaretic acid, tocopherol, tocopherol acetate or natural vitamin E, etc.

The aforesaid crosslinking agent may be a poorly water-soluble aluminum compound, a thermosetting resin such as a polyfunctional epoxy compound, amino resin, phenol resin, epoxy resin, alkyd resin or unsaturated polyester; an isocyanate compound, a block isocyanate compound, an organic crosslinking agent, or an inorganic crosslinking agent such as a metal or metal compound, which may be used alone or in combination.

The aforesaid antiseptic may be ethyl p-hydroxybenzoate, propyl p-hydroxybenzoate or butyl p-hydroxybenzoate.

The aforesaid tackifier may be for example casein, pullulan, agar, dextran, sodium alginate, soluble starch, carboxy starch, dextrin, carboxymethylcellulose, carboxymethylcellulose sodium, methyl cellulose, ethyl cellulose, hydroxyethyl cellulose, polyvinyl alcohol, polyethylene oxide, polyacrylamide, polyacrylic acid, polyvinyl pyrrolidone, carboxy vinyl polymer, polyvinyl ether, maleic acid copolymer, methoxyethylene maleic anhydride copolymer, isobutylene maleic anhydride copolymer, polyethylene imine, polyvinyl alcohol partial saponification product, hydroxypropyl methyl cellulose, xanthan gum or N-vinyl acetamide, etc.

The aforesaid solvent may be for example isopropyl myristate, diisopropyl adipate, crotamiton or mentha oil, etc. Among these, isopropyl myristate is particularly preferred. The blending amounts thereof are preferably 0.2-3 mass %, more preferably 0.5 to 2 mass %, and still more preferably 0.8 to 1.5 mass % based on the total amount of the adhesive layer 1.

The aforesaid pigment may be a certified color such as Red No. 2 (amaranthus), Red No. 3 (erythrocin), Red No. 102 (new coccin), Red No. 104 (1) (phloxine B), Red 105 (1) (rose bengal), Red 106 (acid red), Yellow No.4 (tanrazine), Yellow No. 5 (sunset yellow FCF), Green No. 3 (fast green FCF), Blue No. 1 (brilliant blue FCF) or Blue No. 2 (indigo carmine). The pigment is not particularly limited, but it greatly affects the image of the pharmaceutical preparation, improves user experience and skin rejuvenation feeling.

The aforesaid perfume may be a plant extract such as for example mentha oil, cinnamon oil, clove oil, fennel oil, castor oil, spirit of turpentine, eucalyptus oil, orange oil, lavender oil, lemon oil, rose oil, lemon grass oil, rosemary or sage.

The aforesaid ultraviolet absorber may be for example p-aminobenzoic acid, p-aminobenzoic acid ester, amyl p-dimethyl aminobenzoate, salicylic acid ester, methyl anthranilate, umbelliferone, escurine, benzyl cinnamate, cinnoxate, guaiazulene, urocanic acid, 2-(2-hydroxy-5-methylphenyl) benzotriazol, 4-methoxybenzophenone, 2-hydroxy-4-methoxybenzophenone, dioxybenzone, octabenzone, dihydroxydimethoxybenzophenone, sulisobenzone, benzoresorcinol, octyldimethyl p-aminobenzoate or ethylhexyl p-methoxycinnamate, etc.

The aforesaid inorganic filler may be for example calcium carbonate, magnesium carbonate, a silicate (for example, aluminum silicate, magnesium silicate, etc.), silicic acid, barium sulfate, calcium sulfate, calcium zincate, zinc oxide or titanium oxide.

The aforesaid pH adjuster may be for example acetic acid, formic acid, lactic acid, tartaric acid, oxalic acid, benzoic acid, glycolic acid, malic acid, citric acid, hydrochloric acid, nitric acid, sulfuric acid, sodium hydroxide, calcium hydroxide, monomethylamine, ethylamine, propylamine, dimethylamine, diethylamine, dipropylamine, trimethylamine, triethylamine, tripropylamine, mono-methanol amine, monoethanolamine, mono-propanolamine, dimethanolamine, diethanolamine, dipropanolamine, trimethanolamine, triethanolamine, tripropanolamine, citric acid buffer, phosphoric acid buffer, glycine buffer, acetic acid buffer or other buffer solution, etc.

The pH of the mixed solution (fat) wherein the aforesaid components are blended is preferably such as not to irritate the skin, preferably within the range of 4-8, but more preferably 5-7.

The blending amounts of the aforesaid skin beautifying ingredient, moisturizing ingredient, antioxidant, crosslinking agent, antiseptic, tackifier, solvent, pigment, perfume, ultraviolet absorber, inorganic filler and pH adjuster are not particularly limited, but the total amount of these additives is preferably 5 to 70 mass %, and more preferably 20 to 60 mass % based on the total amount of the adhesive layer 1.

The adhesive layer 1 mentioned above is disposed on the support 2. The support 2 used for the warming patch 10 may be any material which can support the adhesive layer 1, elastic or non-elastic, and may specifically be a fibrous sheet or a resin film. Among these, a fibrous sheet of woven fabric or nonwoven fabric which has moisture permeability is preferred. If such a fibrous sheet support is used, when a patch is applied, sweat which accumulates between an affected body part and the patch can be effectively dispersed, and stuffiness due to sweat and skin irritation can be prevented.

The support 2 may be for example polyurethane, polyester, polypropylene, polyvinyl acetate, polyvinylidene chloride, polyethylene, polyethylene terephthalate, aluminum sheet, a synthetic or natural fiber such as nylon, acryl, cotton, rayon, acetate, or a fibrous sheet of woven or non-woven fabric obtained by compounding these fibers, or a fibrous sheet which is a compound material of these with a film having moisture permeability.

Among these, from the viewpoint of safety, flexibility and elasticity, a fibrous sheet of a woven or nonwoven fabric comprising polyester, polyethylene or polyethylene terephthalate is preferred, and a fibrous sheet of a woven or nonwoven fabric comprising polyethylene terephthalate is more preferred. Even if such a fibrous sheet is thick, it has plasticity, tends to follow the skin and has low skin irritation. By using a fibrous sheet, a patch having moderate self-supporting properties can be obtained.

The thickness of the support 2 is preferably 400-2000 µm, but more preferably 600-1000 µm. If the thickness is less than the aforesaid lower limit, anchoring is insufficient, whereas if on the other hand the aforesaid upper limit is exceeded, the user has an unpleasant feeling when the patch is applied.

The water vapor transmission of the support 2 is preferably 500-6000 g/m²/24hr, more preferably 2000-6000 g/m²/24hr, and still more preferably 3000-5000 g/m²/24hr. If the water vapor transmission of the support is less than the lower limit, it tends to cause stuffiness or a rash, and if on the other hand it exceeds the aforesaid upper limit, the moderate moisturizing effect due to ODT effect tends to be difficult to obtain. The water vapor transmission (moisture permeability) can be measured by the method specified in JIS Z 0208.

Next, referring to the cross-sectional view shown in FIG. 2, a suitable warming patch with a support having a triple layer structure will be described.

A warming patch 20 shown in FIG. 2 has an adhesive layer 1 on one surface of a support 2 having a triple layer structure provided with a thermoplastic resin film 2b and a fibrous sheet 2a of woven or nonwoven fabric disposed on both sides thereof, respectively.

The fibrous sheet 2a may be identical to the fibrous sheet described above. The fibrous sheet 2a preferably has a weave (weight per unit area) of 10-200 g/m², but more preferably a weave of 50-150 g/m². If the weave is less than the aforesaid lower limit, the patch is not easy to handle, it has poor cushioning properties and foreign body sensation tend not to be fully improved. On the other hand, if the weave exceeds the aforesaid upper limit, cushioning properties improve, but the patch tends to become hard overall, and skin irritation increases.

The thickness of the fibrous sheet 2a is preferably 10-200 µm, but more preferably 50-150 µm. If the thickness is less than the aforesaid lower limit, anchoring is poor, whereas if on the other hand the aforesaid upper limit is exceeded, there is an unpleasant feeling when the patch is applied.

The thermoplastic resin film 2b may be a resin film such as for example polyester, polyethylene, polypropylene, polyurethane, polyamide (nylon), polyacetal, polycarbonate, polyvinyl chloride, ABS plastics, polystyrene or (meth)acrylate resin. Among these, a resin film of polyester or polyethylene is particularly preferred.

By arranging the support 2 to have a triple layer structure using the thermoplastic resin film 2b, there is almost no loss of the pharmaceutical agent due to the agent moving into the support 2, and when the patch is stuck to the
skin, a patch 20 which has a moderate ODT (seal bag method) effect and moderate warming effect due to the seal, can be obtained.

The thickness of the thermoplastic resin film 2b is preferably 10-100µm, but more preferably 15-30µm. If the thickness is less than the aforesaid lower limit, self-supporting properties cannot be maintained when the patch is manufactured, barrier properties of the pharmaceutical agent are low and the seal effect is poor. On the other hand, if the aforesaid upper limit is exceeded, the patch cannot mold itself to the application site, the user has an unpleasant feeling, and the application site is easily damaged.

The patch 20 comprising the support 2 of triple layer structure has moderate self-supporting properties, and can support or immobilize body parts affected by, for example, lumbago and muscular pain. It has excellent handling properties, penetration of pharmaceutical agent contained in the adhesive layer 1 from the undersurface (surface opposite to the surface to which the adhesive layer 1 is stuck) of the support 2 can be prevented, and the warming effect of the warming material is remarkably improved due to the seal and heat-retaining effect after applying to the skin.

Further, in the part sealed by the patch 20, the homy layer softens and swells up due to accumulated skin moisture, and percutaneous absorption of the warming material or pharmaceutical agent increases remarkably due to a decrease of skin barrier properties.

The thickness of the support 2 of triple layer structure is preferably 100-1200 µm, but more preferably 300-900 µm. If the thickness is less than the aforesaid lower limit, anchoring properties tend to be poor, whereas if on the other hand, the aforesaid upper limit is exceeded, there is an unpleasant feeling when the patch is applied.

The water vapor transmission of the support 2 of triple layer structure is preferably 500-6000 g/m²/24hr, but more preferably 2000-6000 g/m²/24hr, and still more preferably 3000-5000 g/m²/24hr. If the water vapor transmission of the support is less than the lower limit, rashes or fat deposits tend to occur, and if on the other hand, the aforesaid upper limit is exceeded, the moderate moisturizing effect due to ODT effect is no longer obtained.

In the method of manufacturing the support 2 of triple layer structure, a liquid thermoplastic resin is run between the two fibrous sheets 2a, spread thinly by passing through a roller and laminating before the thermoplastic resin solidifies. The thermoplastic resin is then solidified so that the two fibrous sheets 2a of nonwoven or woven fabric are stuck to the thermoplastic resin. The support 2 of triple layer structure comprising the resin film 2b and fibrous sheets 2a is thereby obtained.

The color of the support is not particularly limited, but since it leads to improvement in user experience or skin rejuvenation feeling, it is preferably colored white, flesh color, yellow, red, orange, green, pink or light blue, etc.

Next, the method of manufacturing the warming patch will be described. In the warming patch 10, 20, the aforesaid adhesive layer 1 is disposed on at least one surface of the support 2, but the method of arranging the adhesive layer 1 on the support 2 is not particularly limited. For example, the ingredients which are generally blended with the adhesive layer, and a mixture of the other ingredients mentioned above which are added if required, are mixed in an agitator to obtain a homogeneous mixture. By coating or spreading this mixture on the support 2, the warming patches 10 and 20 can be obtained.

Here, the warming patch may be provided with one adhesive layer as shown in Figs.1 and 2, or it may be provided with two or more adhesive layers (not shown) unless the skin permeability of the pharmaceutical agent is impaired.

The thickness of the adhesive layer 1 is not particularly limited, but is preferably 20-200 µm. If the thickness of the adhesive layer 1 is less than the aforesaid lower limit, skin permeability of the pharmaceutical agent is poor, whereas if on the other hand it exceeds the aforesaid upper limit, the adhesive agent tends to remain stuck to the skin (residual adhesive agent) after application.

If the patch 10, 20 is to be further provided with a release paper (not shown) on the adhesive layer 1, the patches provided with a release paper can be obtained by coating the aforesaid mixture on the release paper and sticking the support 2 on the coated surface, or by coating the aforesaid mixture on the support 2 and sticking the release paper onto the coated surface.

If the patch 10, 20 is further provided with a release paper, this release paper may be a polyester such as polyethylene terephthalate, a film such as polyvinyl chloride and polyvinylidene chloride, or a laminated film of fine quality paper and polyolefine. In these release papers, if siliconization treatment is given to the surface on the side in contact with the adhesive layer 1, the peeling of the release paper from the patch 10, 20 will be facilitated, which is preferred.

The warming patch 10, 20 thus obtained is preferably cut to a suitable predetermined shape for use. Specifically, it can be fashioned into a suitable shape for application to a body part where it is intended to be used, i.e., chest, back, arms, legs, waist or shoulders. To prevent soiling in storage or
loss of effect due to evaporation of volatile substances, the patch is preferably kept in a sealed bag or container.

### EXAMPLES

Hereafter, the present invention will be described in more detail referring to examples and comparative examples, but the present invention is not be construed as being limited in any way thereby. In the following examples and comparative examples, "%" refers to "mass %" unless otherwise specified.

### Example 1

55.55% purified water, 3% gelatin, 3% polyacrylic acid, 3% polyacrylic acid partially neutralized substance, 2% polyvinyl alcohol, 1% synthetic aluminum silicate, 0.3% magnesium aluminometasilicate, 0.5% polyoxyethylene monostearate, 0.3% disodium ethylenediaminetetraacetate, 0.2% methyl paraben, 20% glycerol, 10% polyethylene glycol, 1.0% 1-menthol and 0.15% capsicum extract (net 10%) were uniformly dispersed and dissolved to obtain a coating solution to be used as the adhesive layer 1. The blending amount of capsaicin in the coating solution was 0.015%. As the polyethylene glycol, Macrogol 400 of molecular weight 400 was used.

Next, the coating solution was spread on the support 2 to 1000 g/m², and the film was stuck thereto. The product was then cut to 10cm x 14cm to obtain a sheet-like warming patch.

The support 2 used in Example 1 was a triple layer support comprising a polyester nonwoven fabric (weave 50 g/m²)/polyethylene film (thickness 15 µm)/polyester nonwoven fabric (weave 50 g/m²), having a water vapor transmission of 3800 g/m²/24hr. The water vapor transmission was measured at a temperature of 40°C and relative humidity of 90% by the
method specified in JIS Z 0208.

### Comparative Example 1

A warming patch was obtained by the same method as in Example 1, except that in the coating solution of Example 1, the blending amount of glycerol was changed to 30%, and polyethylene glycol was not blended.

### Comparative Example 2

A warming patch was obtained by the same method as in Example 1, except that in the coating solution of Example 1, 10% of 70% sorbitol aqueous solution was blended instead of polyethylene glycol.

### Comparative Example 3

A warming patch was obtained by the same method as in Example 1, except that in the coating solution of Example 1, 10% liquid paraffin was blended instead of polyethylene glycol.

### Experiment 1: Residual Irritation Test

A residual irritation test was performed as follows using the warming patches obtained by Example 1 and Comparative Examples 1-3. First, each patch was affixed to the waists of 30 adults (panelists), and left for 8 hours. Next, the patches were peeled off, and all the panelists took a bath after 1 hour.

The residual irritation was evaluated by asking all the panelists to assess the tingling feeling in the bath in the part where the patch had been used, according to the following criteria for tingling intensity:
5 None
4 Slight tingling
3 Tingling
2 Intense, but bearable
1 Unbearable.
and was shown in the average of these results. Table 1 shows the obtained results.

**(TABLE 1)**

| | Ex. 1 | Comp. Ex. 1 | Comp. Ex. 2 | Comp. Ex. 3 |
|---|---|---|---|---|
| Average score | 4.5 | 2.0 | 2.5 | 2.3 |

As is clear from the results of Table 1, residual irritation was fully reduced by the warming patch of Example 1, and comfortable bathing was attained after peeling off the warming patch. On the other hand, when the warming patches of Comparative Examples 1-3 were used, there was a problem of residual irritation, and some panelists could not tolerate the tingling feeling during bathing. The warming patch obtained in Example 1 also had a good warming effect and coolness, and maintained these properties in use.

It was thus found that among numerous residual irritation reducing agents, polyethylene glycol is particularly suitable as an irritation reducing agent which fully reduces residual irritation.

### Industrial Applicability

As described hereintofore, according to the present invention, a warming patch which gives a sufficient warming effect, further increases the durability of the pleasant stimulation accompanying the moderate warming effect and fully reduces residual irritation, is obtained.

## Claims

1. A warming patch comprising a support and adhesive layer disposed on at least one surface of said support,
wherein said adhesive layer contains a warming material, I-menthol, and polyethylene glycol as an irritation reducing agent,
wherein the amount of said I-menthol is 0.5-1.5 mass% based on the total amount of said adhesive layer,
wherein the amount of polyethylene glycol is 5-15 mass% based on the total amount of said adhesive layer,
wherein said warming material is selected from a group comprising capsaicin, dihydroxycapsaicin, capsanthin, capsaicinoid, capsicoside, capsicum extract, capsicum tincture and capsici fructus pulveratus, and
wherein said patch contains 0.01-0.03 mass % of said warming material, based on the total amount of said adhesive layer.

2. The warming patch according to claim 1, wherein the molecular weight of said polyethylene glycol is in the range of 200-4000.

3. The warming patch according to any of claims 1 or 2, wherein said adhesive layer further contains a non-steroidal anti-inflammatory drug.

4. The warming patch according to any of claims 1-3, wherein said support has a triple layer structure comprising a thermoplastic resin film and a fibrous sheet of woven or nonwoven fabric disposed on both sides of said resin film, respectively.

5. The warming patch according to any of claims 1-4, wherein the water vapor transmission of said support is 500-6000g/m²/24hr, and wherein the water vapour transmission is measured by the method specified in JIS (Japan Industrial Standard) Z 0208.

## Patentansprüche

1. Wärmendes Pflaster, umfassend einen Träger und eine Klebstoffschicht, die auf wenigstens einer Oberfläche des Trägers angeordnet ist,
wobei die Klebstoffschicht ein wärmendes Material, I-Menthol und Polyethylenglycol als ein eine Reizung verminderndes Mittel enthält,
wobei die Menge des I-Menthols 0,5-1,5 Massen%, bezogen auf die Gesamtmenge der Klebstoffschicht, beträgt,
wobei die Menge an Polyethylenglycol 5-15 Massen%, bezogen auf die Gesamtmenge der Klebstoffschicht, beträgt,
wobei das wärmende Material ausgewählt ist aus einer Gruppe, umfassend Capsaicin, Dihydroxycapsaicin, Capsanthin, Capsaicinoid, Capsicosid, Capsicum-Extrakt, Capsicum-Tinktur und Capsici fructus pulveratus, und
wobei das Pflaster 0,01-0,03 Massen% des wärmenden Materials, bezogen auf die Gesamtmenge der Klebstoffschicht, enthält.

2. Wärmendes Pflaster gemäß Anspruch 1, wobei das Molekulargewicht des Polyethylenglycols im Bereich von 200-4000 liegt.

3. Wärmendes Pflaster gemäß einem der Ansprüche 1 oder 2, wobei die Klebstoffschicht außerdem ein nicht-steroidales entzündungshemmendes Arzneimittel enthält.

4. Wärmendes Pflaster gemäß einem der Ansprüche 1-3, wobei der Träger eine Dreifachschichtstruktur aufweist, die einen thermoplastischen Harzfilm und eine faserartige Lage aus gewebtem oder nicht gewebtem Stoff umfasst, die auf beiden Seiten des Harzfilms angeordnet ist.

5. Wärmendes Pflaster gemäß einem der Ansprüche 1-4, wobei die Wasserdampfdurchlässigkeit des Trägers 500-6000g/m²/24h beträgt und wobei die Wasserdampfdurchlässigkeit durch das in JIS (Japanischer Industrie-Standard) Z 0208 angegebene Verfahren gemessen wird.

## Revendications

1. Timbre chauffant comprenant un support et une couche adhésive disposée sur au moins une surface dudit support,
ladite couche adhésive contenant un matériau chauffant, du l-menthol, et du polyéthylène glycol comme agent de réduction de l'irritation,
la quantité dudit l-menthol étant de 0,5 à 1,5 % en masse sur la base de la quantité totale de ladite couche adhésive,
la quantité du polyéthylène glycol étant de 5 à 15 % en masse sur la base de la quantité totale de ladite couche adhésive,
ledit matériau chauffant étant sélectionné parmi un groupe comprenant la capsaïcine, la dihydroxycapsaïcine, la capsanthine, le capsaïcinoïde, le capsicoside, l'extrait de capsicum, la teinture de capsicum et la poudre de fruit de capsicum, et
ledit timbre contenant de 0,01 à 0,03 % en masse dudit matériau chauffant, sur la base de la quantité totale de ladite couche adhésive.

2. Timbre chauffant selon la revendication 1, le poids moléculaire dudit polyéthylène glycol se situant dans la plage de 200 à 4 000.

3. Timbre chauffant selon l'une quelconque des revendications 1 ou 2, ladite couche adhésive contenant en outre un médicament anti-inflammatoire non stéroïdien.

4. Timbre chauffant selon l'une quelconque des revendications 1 à 3, ledit support présentant une structure triple couche comprenant un film de résine thermoplastique et une feuille fibreuse de textile tissé ou non-tissé disposée sur respectivement les deux côtés dudit film de résine.

5. Timbre chauffant selon l'une quelconque des revendications 1 à 4, la transmission de vapeur d'eau dudit support étant de 500 à 6 000 g/m²/24 h, et la transmission de vapeur d'eau étant mesurée par l'intermédiaire du procédé spécifié dans la norme JIS (Japan Industrial Standard) Z 0208.
